# EUROPÄISCHE PATENTSCHRIFT

(11) **EP 1 557 196 B1**
(45) Veröffentlichungstag und Bekanntmachung des Hinweises auf die Patenterteilung: **31.03.2010**
(21) Anmeldenummer: 05000575.0
(22) Anmeldetag: 13.01.2005
(51) Int. Cl.: A61N 1/378, A61N 1/05, A61N 1/06, A61N 1/34

(54) **Medizinisches Elektrodensystem**
Medical electrode system
Système d'électrode medicale

(30) Priorität: 20.01.2004 DE 202004000852 U
(43) Veröffentlichungstag der Anmeldung: 27.07.2005
(73) Patentinhaber: Mantsch, Christian, 32427 Minden (DE); Stumpp, Uwe, 78665 Frittlingen (DE)
(72) Erfinder: Mantsch, Christian, 32427 Minden (DE); Stumpp, Uwe, 78665 Frittlingen (DE); Omar-Pasha, Omar, 21029 Hamburg (DE)
(74) Vertreter: Wiebusch, Manfred

(56) Entgegenhaltungen:
- WO-A-01/83029
- US-A- 4 044 774
- US-A- 6 146 380
- US-A1- 2004 044 369
- US-A1- 2004 210 290

## Beschreibung

Die Erfindung betrifft ein Elektrodensystem mit einer implantierbaren, flexiblen Elektrode, insbesondere Epiduralelektrode, mit mindestens einem distalen elektrischen Kontakt. Gemäß einer Weiterentwicklung ist die Elektrode in einem implantierbaren, flexiblen Katheter, insbesondere Epiduralkatheter, angeordnet.

Ein solcher Katheter hat üblicherweise mindestens einen Kanal, der beispielsweise zur Verabreichung von Medikamenten durch den Katheter dient. Mitunter wird aber auch eine Elektrode ohne einen Kanal als Katheter bezeichnet, beispielsweise als Stimulationskatheter. Solche Katheter mit einem distalen elektrischen Kontakt, die keinen Kanal aufweisen und somit keine Katheter im eigentlichen Sinne sind, werden im folgenden als Elektroden bezeichnet.

Katheter sind bekannte medizintechnische Produkte, die für verschiedene Einsatzzwecke in der Diagnostik oder Therapie hergestellt werden. So sind beispielsweise Epiduralkatheter bekannt, die von einem Arzt in den Periduralspalt im Bereich des Wirbelkanals eingeführt werden können, um beispielsweise schmerzstillende Medikamente injizieren zu können. Ein solches Verfahren wird insbesondere zur Behandlung chronischer Schmerzen angewandt. Dabei kann der Katheter beispielsweise eine Zeit von 1 bis 30 Tagen im Körper verbleiben, und die Injektion der Medikamente kann über externe oder implantierte Pumpen erfolgen.

Anstelle von Kathetern werden auch Elektroden in der Therapie chronischer Schmerzen eingesetzt. So sind Elektroden zur Implantierung bekannt, die zur Dauerstimulation des Rückenmarkes oder der Nerven an einen Impulsgenerator angeschlossen werden.

Ferner sind Spezialnadeln bekannt, die an einen Generator für gepulste Hochfrequenz angeschlossen werden. Solche Spezialnadeln und Hochfrequenzgeneratoren werden verwendet, um durch gezielte Reizung von Nerven die Freisetzung von schmerzhemmenden Substanzen im Rückenmark auszulösen und dadurch eine Schmerzbehandlung zu bewirken. Dieser Einsatz dieser Spezialnadeln stößt jedoch häufig an anatomische Grenzen oder wird wegen der Gefahr der Verletzung beim Einführen der Spezialnadeln vermieden.

Aus WO 01/83029 A1 ist ein implantierbarer Neurostimulator mit einer implantierbaren, flexiblen Elektrode bekannt, der induktiv über eine Energieübertragungseinrichtung mit Energie versorgt wird, um seine Batterie zu laden. Die Elektrode weist eine Basiseinheit in Form eines Generators für ein Stimulationssignal auf, welche über mindestens eine Zuleitung elektrisch mit einem distalen elektrischen Kontakt verbunden ist. Die Basiseinheit und die Energieübertragungseinrichtung sind dazu ausgebildet, bei geeigneter Anordnung zueinander, wobei die Energieübertragungseinrichtung nicht implantiert wird, elektromagnetische Energie von der Energieübertragungseinrichtung zur Basiseinheit zu übertragen.

Aus US 2004/0044369 A1 ist ein Herzschrittmacher und Kardioverter-Defibrillator bekannt, bei dem eine Sonde in Form einer Nadel durch die Haut eines Patienten an einen Kontakt des implantierten Geräts geführt wird, um über die Nadel eine Batterie des Geräts aufzuladen. Der Kontakt ist durch eine Gel-artige Isolierung elektrisch isoliert, welche von einem PVC-ähnlichen Material abgedeckt ist.

Aus der EP 1 181 947 A2 ist ein Epiduralkatheter mit mindestens drei in einer Reihe angeordneten Elektroden bekannt. Die Elektroden dienen zur elektrischen Stimulation von Nerven oder des Rückenmarks. Es kann ein Kanal zur Verabreichung von Medikamenten vorgesehen sein, so daß zusätzlich zur elektrischen Stimulation des Rückenmarks oder der Spinalnerven eine Injektion schmerzstillender Medikamente erfolgen kann.

Ein weiterer implantierbarer Epiduralkatheter ist aus der DE 203 08 422 U 1 bekannt. Dieser Katheter ist beispielsweise zur Applikation von gepulster Hochfrequenz zur Nervenstimulation geeignet. Außerdem kann eine Spritze oder eine Medikamentenpumpe angeschlossen werden. Der Katheter weist ein Befestigungselement auf, das zur Befestigung des Katheters an einer Eintrittsstelle in einem Körper dienen kann und durch welches elektrische Zuleitungen und eine Schlauchleitung des Katheters hindurchgeführt sind.

Aufgabe der vorliegenden Erfindung ist es, ein Elektrodensystem der eingangs genannten Art mit einem Katheter oder einer Elektrode zu schaffen, das flexibler einsetzbar ist.

Diese Aufgabe wird erfindungsgemäß durch ein Elektrodensystem der eingangs genannten Art mit den Merkmalen des Patentanspruchs 1 gelöst, bei dem ein Teil des Elektrodensystems durch eine Energieübertragungseinrichtung gebildet wird, an die ein externer Generator für Elektrostimulation abschließbar ist, wobei die Elektrode eine subkutan implantierbare Basiseinheit aufweist, welche über mindestens eine Zuleitung elektrisch mit dem distalen elektrischen Kontakt verbunden ist, und wobei die Basiseinheit und die Energieübertragungseinrichtung dazu ausgebildet sind, bei geeigneter Anordnung zueinander elektromagnetische Energie von dem Generator durch die Energieübertragungseinrichtung zur Basiseinheit zu übertragen. Die Energieübertragungseinrichtung wird nicht implantiert.

Dabei ist gemäß einer Weiterentwicklung die Elektrode in einem implantierbaren, flexiblen Katheter, insbesondere Epiduralkatheter, angeordnet. In diesem Falle weist die Basiseinheit einen subkutan implantierbaren Port auf, in dem eine Zugangsöffnung des Katheters angeordnet ist.

Nach dem Anlegen der Elektrode bzw. des Katheters kann dieser vollständig mitsamt der Basiseinheit nahe der Eintrittsstelle in den Körper unterhalb der Haut implantiert werden. Der unter der Haut verborgene Katheter ist für den Patienten weniger hinderlich. Außerdem ist das Infektionsrisiko und die Gefahr von Komplikationen verringert. Das erfindungsgemäße Elektrodensystem erlaubt es daher beispielsweise, nach einer ambulanten Stimulationsbehandlung eines Patienten die Elektrode oder den Katheter im Körper des Patienten zu belassen und den Patienten nach Hause zu entlassen, bis beispielsweise nach einigen Tagen oder Wochen eine erneute Stimulation notwendig wird. Für diese erneute Stimulation wird dann der bereits implantierte Katheter verwendet.

Das Elektrodensystem mit dem Katheter stellt eine Weiterentwicklung dar, die ferner auch gegenüber einem herkömmlichen implantierbaren Katheter oder Stimulationskatheter einen vergrößerten Einsatzbereich verschließt. Wird der Katheter beispielsweise als Epidurallcatheter in den Bereich des Wirbelkanals eingelegt, so kann neben der Stimulation des Rückenmarks oder der Spinalnerven durch beispielsweise einen gepulsten Hochfrequenzstrom nach Bedarf zusätzlich die Injektion eines schmerzstillenden Medikaments durch den Port und die Zugangsöffnung des Katheters erfolgen. Der implantierbare Port kann beispielsweise ein Septum aufweisen oder in Form eines Septums ausgeführt sein, das von extern mit einer Injektionsnadel erreichbar ist. Der erfindungsgemäße Katheter hat also in der Anwendung deutliche Vorteile gegenüber einem herkömmlichen Katheter und verschließt so einen vergrößerten Einsatzbereich.

Erfindungsgemaß weist bei dem Elektrodensystem mit Elektrode oder Katheter die Basiseinheit einen subkutan implantierbaren Port auf, die Energieübertragungseinrichtung weist eine in den Port einführbare Sonde mit mindestens einem Sondenkontakt auf, und in dem Port ist mindestens ein mit dem distalen elektrischen Kontakt verbundenes elektrisches Kontaktelement zur Herstellung einer elektrischen Verbindung mit dem Sondenkontakt angeordnet.

Es kann beispielsweise im oben beschriebenen Fall einer bereits implantierten Elektrode oder eines bereits implantierten Katheters für eine erneute Stimulation eine Sonde in Form einer Nadel in den Port eingeführt werden, um erneut einen Stimulationsstrom zu applizieren. Dies ist sehr viel einfacher durchzuführen und ist weniger aufwendig als beispielsweise eine erneute Implantation einer Elektrode oder eines Katheters. Das Elektrodensystem mit der Sonde hat also in der Anwendung deutliche Vorteile gegenüber einer herkömmlichen implantierbaren Elektrode.

Erfindungsgemäß weist die Sonde außerdem ein Kupplungselement auf, und der Port der Elektrode oder des Katheters weist eine Ankuppelvorrichtung für das Kupplungselement auf. Diese stellt beispielsweise ein Mittel zur mechanischen Verankerung der Sonde an dem Port dar. Dabei kann es sich beispielsweise um eine mechanische Halterung oder Verriegelung handeln, beispielsweise einen Klickverschluß. Durch die Ankuppelvorrichtung und das Kupplungselement kann insbesondere ein sicherer Kontakt zwischen dem Sondenkontakt und dem elektrischen Kontaktelement hergestellt werden. Wenn die Sonde an dem Port gehalten wird, wird außerdem beispielsweise das Applizieren von Pulsen zur Nervenstimulation erleichtert.

Vorzugsweise wird das Kupplungselement der Sonde durch einen Gewindeansatz gebildet. Vorzugsweise wird die Ankuppelvorrichtung durch ein Gewindeelement gebildet. In dieses kann beispielsweise die Sonde mit dem Gewindeansatz eingeschraubt werden.

In einer bevorzugten Ausführungsform der Elektrode oder des Katheters weist der Port ein Septum zum Durchstechen mit einer Nadel auf. Beispielsweise wird die Sonde durch die Nadel gebildet. Alternativ nimmt die Nadel die Sonde auf, und die Sonde ist aus der Nadel vorschiebbar. Dabei ist das elektrische Kontaktelement innerhalb des Ports so angeordnet, daß eine elektrische Verbindung zu mindestens einem Sondenkontakt der Sonde herstellbar ist. Außerdem ist beispielsweise im Falle der Ankuppelvorrichtung auch die Ankuppelvorrichtung so in bezug auf das Septum angeordnet, daß die Sonde an die Ankuppelvorrichtung ankoppelbar ist. Ein Septum hat den Vorteil, daß der Innenraum des Ports sowohl mit als auch ohne eingestochene Nadel gegenüber der Umgebung isoliert und abgeschlossen ist.

Vorzugsweise ist ein Führungselement am Port vorgesehen, welches die Sonde zum elektrischen Kontaktelement und gegebenenfalls zur Ankuppelvorrichtung führt.

Im Falle des Katheters weist vorzugsweise die Sonde einen Zugangskanal für die Zugangsöffnung des Katheters auf. Dabei ist vorzugsweise der Sondenkontakt gegenüber dem Inneren des Zugangskanals elektrisch isoliert.

Vorzugsweise sind die Sonde und der Port so gestaltet, daß der Sondenkontakt und das elektrische Kontaktelement gegenüber dem Zugangskanal und der Zugangsöffnung des Katheters abdichtbar sind.

In einer Ausführungsform des Elektrodensystems mit Elektrode oder Katheter die nicht Teil der vorliegenden Erfindung bildet, ist in der Basiseinheit ein Übertrager angeordnet, der über die mindestens eine Zuleitung mit dem mindestens einen distalen elektrischen Kontakt verbunden ist, und der Übertrager und die Energieübertragungseinrichtung sind dazu ausgebildet, Energie induktiv von der Energieübertragungseinrichtung zur Basiseinheit zu übertragen.

Beispielsweise kann das Elektrodensystem einen Katheter, insbesondere Epiduralkatheter aufweisen, der im distalen Bereich mindestens zwei elektrische Kontakte aufweist, deren Zuleitungen im Katheter angeordnet sind. Dabei sind die elektrischen Kontakte über die Zuleitungen mit dem subkutan implantierbaren Übertrager verbunden, und der Katheter weist im proximalen Bereich einen subkutan implantierbaren Port auf.

Nach dem Anlegen der Elektrode oder des Katheters kann dieser wiederum vollständig mitsamt dem Übertrager und dem Port nahe der Eintrittsstelle in den Körper unterhalb der Haut implantiert werden. Bei der späteren Verwendung der Elektrode oder des Katheters beispielsweise zur Stimulation des Rückenmarks oder von Nerven ist durch die verschlossene Haut ein verringertes Infektionsrisiko gegeben, und die Gefahr von Komplikationen ist verringert. Außerdem ist der unter der Haut verborgene Katheter für den Patienten einfacher in der Handhabung.

Energie, beispielsweise in Form von Pulsen zur Stimulation, kann von der Energieübertragungseinrichtung in Form eines externen Gerätes beispielsweise induktiv auf den Übertrager übertragen werden. Alternativ kann der Übertrager induktiv mit Energie versorgt werden und selbst die Pulse zur Nervenstimulation erzeugen.

Der Übertrager kann auch einen Pulsgenerator zur Stimulation oder Dauerstimulation von Nerven aufweisen.

Vorzugsweise weist der Übertrager eine Spule auf. Bei dieser kann es sich insbesondere um eine Sende- und Empfangsspule handeln. Indem die Spule auch als Sendespule einsetzbar ist, können von dem Katheter Signale an ein Gerät außerhalb des Körpers gesendet werden.

In einer bevorzugten Ausführungsform sind der Übertrager und der Port in einem flachen, subkutan implantierbaren Gehäuse angeordnet. Die Kombination des Ports und des Übertragers der Basiseinheit in einem Gehäuse erleichtert ihre Auffindung, wenn beispielsweise ein Medikament zugespritzt werden soll. Das Gehäuse kann außerdem beispielsweise die Spule des Übertragers aufnehmen und eine Stützfläche für die Zuspritzkammer oder den Port bilden, und so die Handhabung bei der Einführung einer Injektionsnadel in den Port erleichtern.

Der Übertrager kann wahlweise eine Vorrichtung zur Energiespeicherung enthalten. Mit dieser kann beispielsweise eine Energieversorgung einer Medikamentenpumpe erfolgen.

Eine solche zwischen dem Port und dem Katheter angeordnete, ebenfalls implantierbare Medikamentenpumpe kann wahlweise bei allen Ausführungsformen mit Katheter vorgesehen sein. Dadurch ist eine gleichmäßige Verteilung der Medikamentengabe über einen längeren Zeitraum erreichbar.

Ferner ist wahlweise an dem Port eine Zuspritzkammer für den Katheter angeordnet. Dadurch wird das Zuspritzen von Medikamenten in den Port erleichtert, und die Zuspritzkammer kann außerdem als Vorratskammer für eine implantierbare Medikamentenpumpe dienen.

Im folgenden werden weitere vorteilhafte Merkmale beschrieben, die sowohl bei der ersten Ausführungsform mit der Sonde als auch bei der zweiten Ausführungsform mit dem Übertrager vorgesehen sein können.

Im Falle des Katheters ist eine distale Öffnung des Katheters bevorzugt zwischen zweien der elektrischen Kontakte angeordnet. Eine solche Anordnung ermöglicht es, denselben Bereich sowohl mit Medikamenten als auch mit elektrischer Stimulation zu behandeln, ohne daß die Lage des Katheters verändert werden muß. Außerdem hat die Nähe der Kontakte zu der distalen Öffnung des Katheters den Vorteil, daß während des Anlegens des Katheters die Lage des Katheters durch eine Teststimulation mit geringerer Spannung und Frequenz überprüft werden kann.

Weiterhin ist sowohl im Falle des Katheter als auch im Falle der Elektrode vorteilhaft, daß die Kontakte einen ausreichenden Röntgenkontrast bieten, um eine Positionierung des Katheters oder der Elektrode mit Röntgenkontrolle ohne Kontrastmittelgabe zu ermöglichen.

Weitere vorteilhafte Ausgestaltungen der Erfindung ergeben sich aus den Unteransprüchen.

In einer bevorzugten Ausführungsform weist die Elektrode oder der Katheter im distalen Bereich einen Temperatursensor auf, dessen Anschlußleitungen in der Elektrode bzw. dem Katheter angeordnet sind oder der über die Anschlußleitungen mit der Basiseinheit verbunden ist.

Bei der Ausführungsform mit Übertrager kann dieser dazu ausgebildet sein, ein von dem Temperatursensor bewirktes Signal zu erfassen.

Wird ein Katheter mit Temperatursensor beispielsweise als Epiduralkatheter im Bereich des Wirbelkanals angelegt und eine gepulster Hochfrequenzstrom über zwei Elektroden an das Rückenmark oder die Spinalnerven appliziert, kann mittels des Temperatursensors eine durch die Stimulation verursachte oder durch die zugeführte elektrische Energie bewirkte Temperaturerhöhung überwacht werden. Zur Überwachung der Temperatur kann beispielsweise ein externes Gerät über die Sonde angeschlossen sein, oder der Übertrager kann Signale an ein externes Gerät senden, und es kann eine externer oder im Übertrager angeordneter Überwachungsschaltkreis vorgesehen sein, der beim Erreichen einer Höchsttemperatur von beispielsweise 42° C die Stimulation automatisch abschaltet oder zeitweise unterdrückt, so daß eine thermische Schädigung des Gewebes vermieden werden kann.

Durch die Überwachung der Gewebetemperatur mittels Temperatursensor können Nerven oder das Rückenmark mit Pulsen oder gepulster Hochfrequenz behandelt werden, deren Parameter innerhalb größerer Grenzen variiert werden können, ohne daß eine thermische Schädigung des Gewebes zu befürchten ist. Die Intensität der Stimulation kann daher optimal an die Bedürfnisse des Patienten angepaßt werden. So kann energiereiche, gepulste Hochfrequenz auch bei häufiger oder länger andauernder Stimulation eingesetzt werden, da so akkumulierende Temperaturerhöhungen zuverlässig überwacht werden können. Auch wenn sich durch die Anlagerung von Gewebe oder Veränderung der lokalen Gewebestruktur die elektrischen Parameter des Gewebes ändern und durch erhöhten Leitungsverlust mehr Energie eingebracht wird, kann durch die Temperaturüberwachung eine Schädigung vermieden werden.

Vorzugsweise ist der Übertrager dazu ausgebildet, ein elektrisches Signal von den elektrischen Kontakten zu empfangen. Dadurch kann, wie auch bei der Ausführungsform mit der Sonde, beispielsweise eine Messung eines Nervenerregungspotentials erfolgen.

Eine Stimulation kann mit beliebigen geeigneten elektrischen Signalen oder Spannungen erfolgen. Vorzugsweise sind jedoch die Basiseinheit und, je nach Ausführungsform, der Übertrager oder das elektrische Kontaktelement und der Sondenkontakt dazu ausgebildet, Radiofrequenz auf die Zuleitung zu übertragen. Somit kann eine Stimulation mittels Hochfrequenzpulsen stattfinden. Die Radiofrequenz kann beispielsweise in Form von Pulsen zur Nervenstimulation übertragen werden. Dabei kann es sich insbesondere um Hochfrequenzpulse handeln. So kann beispielsweise bei einer Epiduralelektrode oder einem Epiduralkatheter durch eine Reizung der Nerven innerhalb des Wirbelkanals in vielen Fällen eine Behandlung oder Reizung von Nervengeflechten vor der Wirbelsäule oder in gefährlichen Bereichen mit Spezialnadeln vermieden werden, und es können auch Nerven mit Hochfrequenzpulsen behandelt werden, die ansonsten dafür nicht zugänglich wären.

Im folgenden werden bevorzugte Ausführungsbeispiele anhand der Zeichnung näher erläutert.

Es zeigen:
- Figur 1: eine schematische Darstellung eines ersten Ausführungsbeispiels eines Elektrodensystems mit einem Epiduralkatheter mit einem implantierbaren Port sowie einer Nadel, die eine Sonde aufnimmt;
- Figur 2: eine schematische Darstellung eines Längsschnitts durch die Spitze des Epiduralkatheters;
- Figur 3: eine schematische, perspektivische Ansicht des Ports mit eingeführter Sonde;
- Figur 4: eine schematische Darstellung eines zweiten Ausführungsbeispiels eines Elektrodensystems mit einem Epiduralkatheter das nicht Teil der vorliegenden Erfindung bildet, mit einem Übertrager mit einer Spule in einem flachen, subkutan implantierbaren Gehäuse sowie ein externes Gerät mit einer Antenne;
- Figur 5: das Gehäuse und den Übertrager mit der Spule in einer anderen Ansicht;
- Figur 6: eine schematische Darstellung eines Längsschnittes durch die Spitze des Epiduralkatheters aus Figur 4; und
- Figur 7: einen Querschnitt durch den Katheter aus Figur 6.

Aus Gründen der Übersichtlichkeit sind die Zeichnungen nicht maßstabsgetreu.

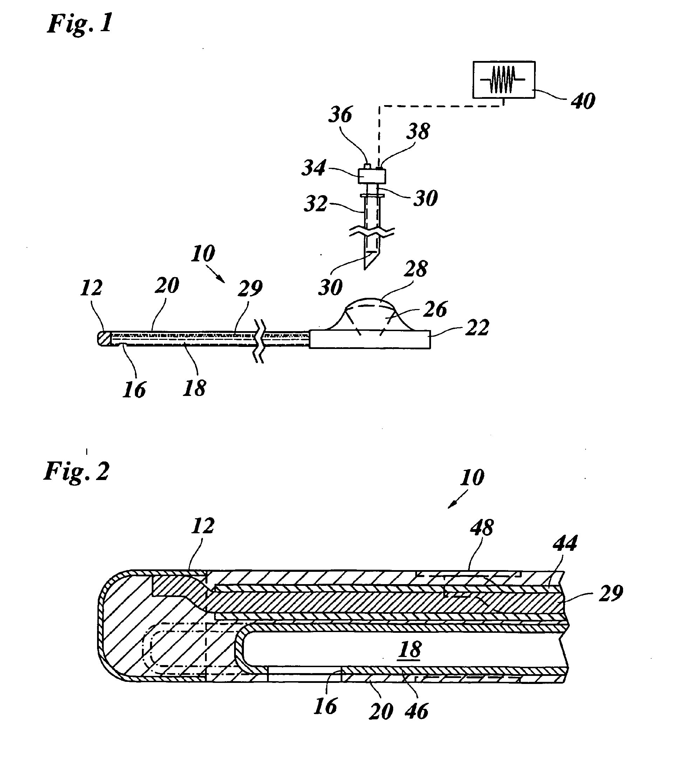

Der in Figur 1 gezeigte Epiduralkatheter 10 weist im distalen Bereich einen elektrischen Kontakt 12 auf, der eine Kappe bildet, die das Ende des Katheters 10 umhüllt. Der Kontakt 12 ist schraffiert dargestellt. Neben dem Kontakt 12 ist eine seitliche Öffnung 16 einer Schlauchleitung 18 des Katheters angeordnet. Die Kante des elektrischen Kontakts 12 schließt bündig mit einer aus Silikonkautschuk gefertigten Hülle 20 des Katheters 10 ab. Der Außendurchmesser der Hülle 20 beträgt 1,33 mm, entsprechend einer Maßangabe von 4 French. In Längsrichtung des Katheters 10 hat der Kontakt 12 eine Ausdehnung, die in etwa dem Außendurchmesser der Hülle 20 entspricht.

Am proximalen Ende ist der Katheter 10 nahtlos mit einer Basiseinheit in Form eines flachen Gehäuses 22 verbunden. Die obere Wand des Gehäuses 22 weist eine Ausbuchtung auf, in der ein Port 26 ausgebildet ist, dessen obere Wandung durch ein Anstechseptum 28 gebildet wird. Über das Anstechseptum 28 ist der Port 26 von extern beispielsweise mittels einer Injektionsnadel zugänglich. Das Anstechseptum 28 ist in bekannter Weise so gefertigt, daß seine Wand eine hinreichende Dichte und Elastizität aufweist, um sich nach dem Herausziehen einer zuvor hindurchgesteckten Injektionsnadel wieder zuverlässig zu verschließen.

Im Inneren des Katheters 10 verläuft eine elektrische Zuleitung 29 für den elektrischen Kontakt 12 parallel zu der Schlauchleitung 18 in der Hülle 20 und ist wie diese gestrichelt angedeutet. Der Aufbau des Katheters 10 wird weiter unten anhand der Figur 2 näher erläutert.

In bekannter Weise ist in der Schlauchleitung ein nicht dargestellter steriler Führungsdraht angeordnet, der zum Verschieben des Katheters 10 an die gewünschte Position im Wirbelkanal dient und danach zurückgezogen wird. Eine Einführungsöffnung für den Führungsdraht wird vor dem Implantieren des Gehäuses 22 verschlossen. Der Führungsdraht ist im Bereich seines vorderen Endes leicht biegsam.

Eine in Figur 1 dargestellte Sonde 30 ist in einer Nadel 32 aufgenommen und verschieblich darin geführt. An ihrem hinteren Ende ist sie mit einem Adapter 34 verbunden, an der ein Anschluß 36 für eine Spritze oder eine Medikamentenpumpe und ein elektrischer Anschluß 38 angeordnet sind. Die Sonde 30 bildet mit dem Adapter 34 eine Energieübertragungseinrichtung für den Katheter 10. Der elektrische Anschluß 38 ist direkt oder über einen nicht gezeigten Adapter zum Anschluß an einen Pulsgenerator 40 geeignet, der einen gepulsten Hochfrequenzstrom erzeugt. Bei dem Pulsgenerator 40 kann es sich beispielsweise um das Gerät N50 der Firma Stryker How Medica, das Gerät RFG-3C+ der Firma Radionics oder das Gerät Neurotherm der Firma RDG Medical handeln.

Figur 2 zeigt die Spitze des Katheters 10 in einer Darstellung als Längsschnitt. Parallel zur Schlauchleitung 18 verläuft die elektrische Zuleitung 29, die an den elektrischen Kontakt 12 innen angelötet ist. Die elektrische Zuleitung 29 weist eine Isolierung 44 auf und verläuft innerhalb eines verdickten Bereiches der Wand der Hülle 20. Die Schlauchleitung 18 wird durch eine zusätzliche innere, schlauchförmige Schicht 46 in der Hülle 20 gebildet. Die Hülle 20 umschließt dabei sowohl den von der inneren Schicht 46 gebildeten Schlauch als auch die Isolierung 44. Die innere Schicht 46 ist durch die Hülle 20 von dem Kontakt 12 isoliert. Mindestens an der Öffnung 16, die die Schicht 46 und die Hülle 20 durchstößt, ist die innere Schicht 46 dicht mit der Hülle 20 verbunden. Sie kann jedoch auch Teil einer aus zwei oder mehreren Schichten aufgebauten Hülle des Katheters sein.

Der von der inneren Schicht 46 geformte Schlauch endet jenseits der Öffnung 16. Er kann jedoch auch, wie durch strichpunktierte Linien angedeutet ist, bis in die von dem distalen Kontakt 12 gebildete Kappe hineinragen.

Alternativ oder zusätzlich zu dem kappenförmigen elektrischen Kontakt 12 kann jedoch auch ein gestrichelt angedeuteter ringförmiger Kontakt 48 in der Nähe der seitlichen Öffnung 16 vorgesehen sein. Dabei kann alternativ zur seitlichen Öffnung 16 eine Öffnung am Ende des Katheters 10 vorgesehen sein.

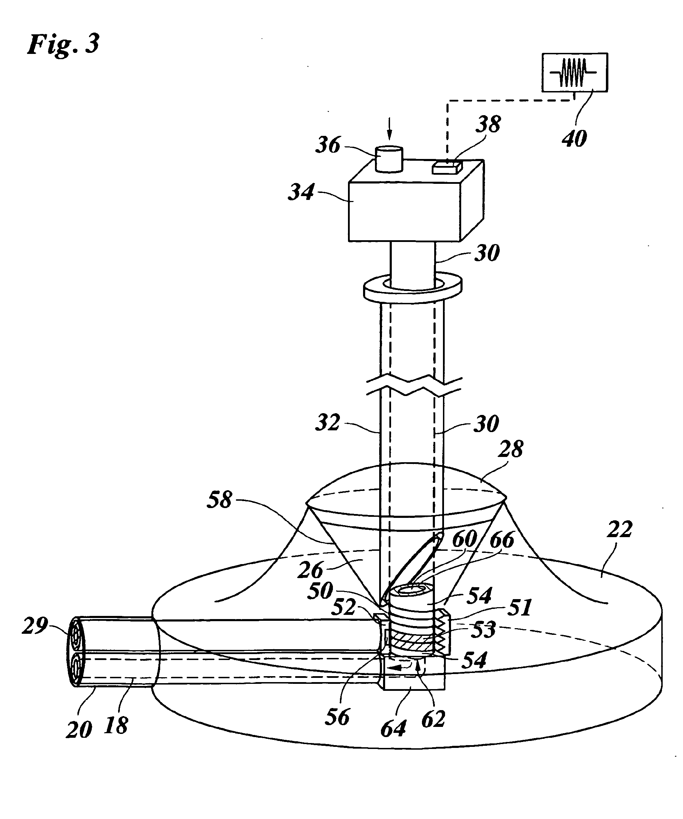

In Figur 3 ist die Nadel 32 durch das Anstechseptum 28 hindurchgestochen, und die Sonde 30 wurde durch die Nadel 32 in den Port 26 eingeführt. Die Sonde 30 ist im unteren Bereich aufgeschnitten dargestellt.

Die Sonde 30 weist an ihrem unteren Ende einen Gewindeansatz 50 auf, der in eine im Gehäuse 22 ausgebildete Fassung mit einem Gewindeelement 51 und einem Gegenstück 52 eingeschraubt ist.

Im Bereich des Gewindeansatzes 50 weist die Sonde 30 einen elektrisch leitfähigen Bereich auf, der einen schraffiert dargestellten Sondenkontakt 53 bildet und sich beispielsweise über eine Höhe von zwei Gewindegängen erstreckt. Oberhalb und unterhalb des Sondenkontakts weist die Außenwand der Sonde einen Isolationsmantel 54 auf.

Der Gewindeansatz 50 mit dem Sondenkontakt 53 ist zwischen dem Gewindeelement 51 und dem Gegenstück 52 gehalten, welches ein elektrisches Kontaktelement 56 aufweist. Das elektrische Kontaktelement 56 erstreckt sich über einen begrenzten Höhenbereich des Gegenstücks 52 und liegt etwa auf der Höhe, an der sich der Sondenkontakt 53 befmdet, wenn die Sonde 30 bis zu einem Anschlag in die Fassung eingeschraubt ist. Auf diese Weise ist eine elektrische Verbindung zwischen dem Sondenkontakt 53 und dem mit der elektrischen Zuleitung 29 verbundenen elektrischen Kontaktelement 56 hergestellt. Zugleich ist die Sonde 30 mechanisch an das Gehäuse 22 des Ports 26 angekoppelt. Dabei bildet der Gewindeansatz 50 ein Kupplungselement der Sonde 30, und die Fassung mit dem Gewindeelement 51 und dem Gegenstück 52 bildet eine Ankuppelvorrichtung des Ports 26.

Am Port 26 ist unterhalb des Anstechseptums 28 ein Konus 58 angeordnet, der ein Führungselement für die Sonde 30 bildet und so das Plazieren des Gewindeansatzes 50 in dem Port 26 erleichtert. Durch Drehen der Sonde 30 in der Nadel 32 wird dann der Gewindeansatz 50 in die Fassung mit dem Gewindeelement 51 eingeschraubt.

Der Sondenkontakt 53 ist mit dem elektrischen Anschluß 38 des Adapters 34 verbunden. Auf diese Weise wird über die Energieübertragungseinrichtung, die Sonde 30 und den Adapter 34, eine elektrische Verbindung zwischen dem Pulsgenerator 40 und dem elektrischen Kontakt 12 des Katheters 10 hergestellt. Der Kontakt 12, die Zuleitung 29, das elektrische Kontaktelement 56, der Sondenkontakt 53 und die Sonde 30 mit dem Adapter 34 sind sowohl zur Applikation von Pulsen zu einer Teststimulation von Nerven oder des Rückenmarks geeignet, die beispielsweise eine Spannung im Bereich von 0 - 12 V mit einer Frequenz im Bereich von 50 bis 150 Hz und einer Pulsweite im Bereich von 150 bis 400 Mikrosekunden aufweisen können, als auch zu Applikation von gepulster Hochfrequenz mit einer Spannung beispielsweise im Bereich von 20 bis 30 V und einer gepulsten Frequenz von beispielsweise 50 kHz oder 500 kHz mit einer Pulsweite von 20 Millisekunden. Die angegebenen Zahlenwerte sind lediglich Beispiele zur Verdeutlichung der Einsatzbreite des Katheters.

Im Inneren der Sonde 30 verläuft ein Zugangskanal 60, der am unteren Ende geöffnet ist und am oberen Ende mit dem Anschluß 36 des Adapters 34 verbunden ist. Das untere Ende des Zugangskanals 60 ist bei mit dem Gewindeelement 51 verschraubtem Gewindeansatz 50 vor einer Zugangsöffnung 62 eines Verbindungselements 64 des Ports 26 angeordnet. Über das Verbindungselement 64 ist die Zugangsöffnung 62 mit der Schlauchleitung 18 des Katheters 10 und somit mit der Öffnung 16 verbunden.

Die Wand des Zugangskanals 60 weist eine Isolationsschicht 66 auf, die am unteren Ende der Sonde 30 mit dem Isolationsmantel 54 verbunden ist, so daß der Zugangskanal 60 gegenüber dem Sondenkontakt 53 und dessen Zuleitung zum elektrischen Anschluß 38 elektrisch isoliert ist. Die Sonde 30 ist im gezeigten Beispiel so weit in die Fassung mit dem Gewindeelement 51 eingeschraubt, daß sie dicht an das Verbindungselement 64 anschließt. Dadurch sind der Sondenkontakt 53 und das elektrische Kontaktelement 56 gegenüber dem Zugangskanal 60 und der Zugangsöffnung 62 des Katheters 10 abgedichtet, so daß eine sich im Zugangskanal 60 und der Schlauchleitung 18 befindende Flüssigkeit nicht in Kontakt zu dem Sondenkontakt 53 gerät. Wahlweise kann die Abdichtung beispielsweise auch am Außenumfang des Gewindeansatzes 50 unterhalb des elektrischen Kontaktelements 56 erfolgen.

Die Sonde 30 und der Katheter 10 erlauben es, nach der Implantation des Katheters 10 bei einer späteren, weiteren Behandlung des Patienten mit einem Einführen der Nadel 32 mit der Sonde 30 in den Port 26 auszukommen, ohne daß weitere Operationen notwendig wären. Außerdem hat die Nähe des Kontaktes 12 zu der distalen Öffnung 16 des Katheters 10 den Vorteil, daß während des Anlegens des Katheters 10 die Lage des Katheters durch eine Teststimulation mit geringerer Spannung und Frequenz überprüft werden kann. Ein weiterer Vorteil ist, daß der Kontakt einen ausreichenden Röntgenkontrast bietet, um eine Positionierung des Katheters mit Röntgenkontrolle ohne Kontrastmittelgabe zu ermöglichen. Ein Vorteil ist außerdem, daß derselbe Bereich sowohl mit Medikamenten als auch mit elektrischer Stimulation behandelt werden kann, ohne daß die Lage des Katheters verändert werden muß. Über den Anschluß 36 kann somit beispielsweise ein Medikament in den Katheter 10 eingespritzt werden.

Wahlweise kann beispielsweise auf eine Abdichtung der Verbindung zwischen dem Zugangskanal 60 und der Schlauchleitung 18 durch das Verbindungselement 64 oder durch die Fassung mit dem Gewindeelement 51 auch verzichtet werden, da das Innere des Ports 26 durch das Anstechseptum 28 jedenfalls stets gegenüber dem Körper des Patienten abgedichtet ist und ein Kontakt der Flüssigkeit zum elektrischen Kontaktelement 56 gegebenenfalls hinnehmbar ist.

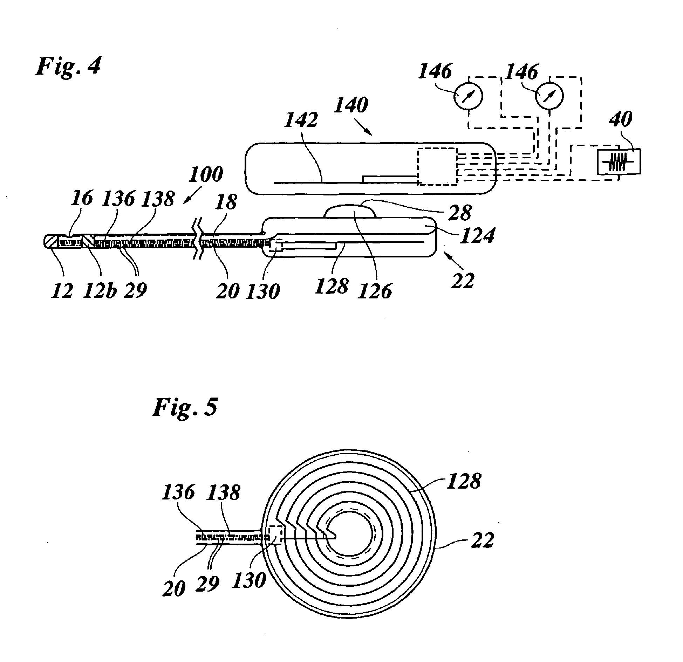

Figur 4 zeigt einen anderen Epiduralkatheter 100, der ähnlich wie der Katheter 10 aufgebaut ist, wobei die Unterschiede im folgenden beschrieben werden. Ähnliche und vergleichbare Elemente sind mit den gleichen Bezugsziffern gekennzeichnet.

Der Katheter 100 weist im distalen Bereich neben dem distalen Kontakt 12 einen proximalen Kontakt 12b auf, zwischen denen die seitliche Öffnung 16 der Schlauchleitung 18 angeordnet ist. Die Kontakte 12 und 12b sind schraffiert dargestellt. Der proximale Kontakt 12b bildet ein ringförmiges Band, das den Katheter 100 umschließt. Die Kanten der Kontakte 12 und 12b schließen bündig mit der Hülle 20 des Katheters 100 ab.

In Längsrichtung des Katheters 100 haben die Kontakte 12 und 12b eine Ausdehnung, die in etwa dem Außendurchmesser der Hülle 20 entspricht. Die Kontakte 12 und 12b sind zueinander um etwa 4 mm in Längsrichtung des Katheters 100 versetzt.

Am proximalen Ende ist der Katheter 100 nahtlos mit einer Basiseinheit in Form des Gehäuses 22 verbunden. Das Gehäuse 22 enthält im oberen Bereich eine Zuspritzkammer 124, die mit der Schlauchleitung 18 verbunden ist. Die obere Wand der Zuspritzkammer 124 weist eine Ausbuchtung auf, die einen Port 126 in Form des Anstechseptums 28 bildet. Über den Port 126 ist die Zuspritzkammer des implantierten Katheters von extern beispielsweise mittels einer Injektionsnadel zugänglich. Die Ausbuchtung stellt somit eine Zugangsöffnung für den Katheter 100 dar.

Im unteren Bereich des Gehäuses 22 ist spiralförmig eine Spule 128 angeordnet, wie in Figur 5 deutlicher zu erkennen ist. Bei der Spule handelt es sich um eine Sendeund Empfangsspule, die mit einem Übertrager 130 verbunden ist. Der Übertrager 130 hat verschiedene Funktionen, die im folgenden noch erläutert werden.

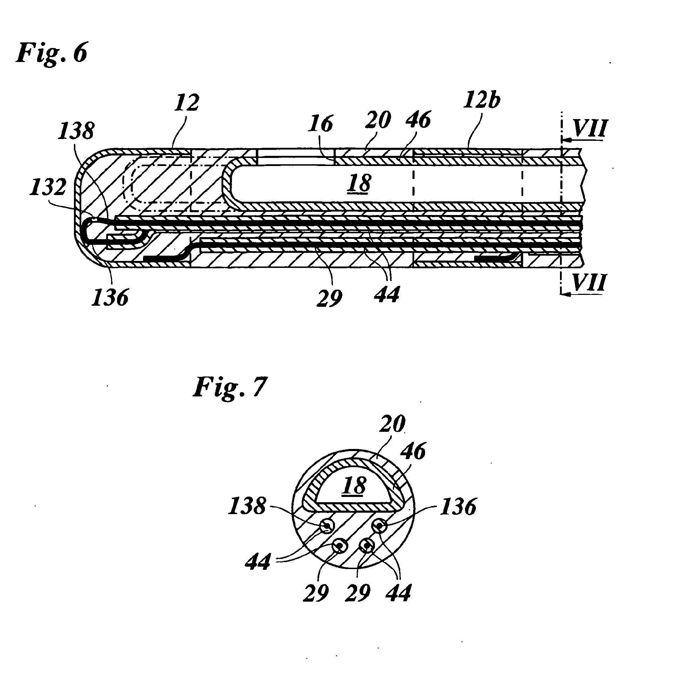

Im Inneren des Katheters 100 ist ein Thermoelement 132 (Figur 6) thermisch mit dem distalen Kontakt 12 verbunden. Die elektrischen Zuleitungen 29 für die elektrischen Kontakte 12 und 12b sowie Anschlußdrähte 136 und 138 des Thermoelements 132 verlaufen parallel zu der Schlauchleitung 18 in der Hülle 20 und sind wie diese gestrichelt angedeutet. Das Thermoelement 132 ist vom Typ Nickel-Chrom/Nickel mit dem Anschlußdraht 136 aus Nickel-Chrom und dem Anschlußdraht 138 aus Nickel. Der weitere Aufbau des Katheters 100 wird weiter unten anhand der Figuren 6 und 7 näher erläutert werden.

Die elektrischen Zuleitungen 29 und die Anschlußdrähte 136, 138 sind mit dem Übertrager 130 verbunden. Der Übertrager ist dazu ausgebildet, Ströme und/oder Spannungen zu messen. Der Übertrager 130 kann beispielsweise eine an den Anschlußleitungen 136 und 138 des Thermoelements anliegende Thermospannung messen und so die Temperatur am distalen Ende des Katheters 100 überwachen. Der Übertrager 130 kann außerdem zwischen den elektrischen Kontakten 12 und 12b anliegende Potentiale messen, die beispielsweise Informationen über den Erregungszustand von Nervenwurzeln oder des Rückenmarks geben können.

Der Übertrager 130 wird über eine Energieübertragungseinrichtung in Form eines externen Gerätes 140 angesteuert, das eine Antenne 142 aufweist, die mit der Sende- und Empfangsspule 128 des Übertragers 130 zusammenwirkt. An das externe Gerät 140 sind der Pulsgenerator 40 sowie Anzeigevorrichtungen 146 anschließbar.

Der Pulsgenerator 40 erzeugt einen gepulsten Hochfrequenzstrom. Die Hochfrequenzpulse werden von der Antenne 142 induktiv auf die Spule 128 übertragen und von dem Übertrager 130 auf die Zuleitungen 29 zu dem elektrischen Kontakten 12 und 12b gegeben. Die Kontakte 12 und 12b, die Zuleitungen 29 sowie der Übertrager 130 und die Spule 128 sind sowohl zur Applikation von Pulsen zu einer Teststimulation von Nerven oder des Rückenmarks als auch zur Applikation von gepulster Hochfrequenz geeignet, wobei die möglichen Spannungs- und Frequenzwerte beispielsweise den beim ersten Ausführungsbeispiel genannten Bereichen entsprechen können.

In Pausen zwischen den Pulsen sowie in Zeiten, bei denen keine Stimulation stattfindet, kann der Übertrager 130 über die Spule 128 Signale an das externe Gerät 140 senden, das diese über seine Antenne 142 empfängt. Dabei können sowohl Informationen über die vom Temperatursensor gemessene Temperatur übertragen werden, als auch Informationen über elektrische Signale, die der Übertrager 130 von den elektrischen Kontakten 12 und 12b empfängt. Ferner können, beispielsweise zu Steuerungszwecken, weitere Signale von dem Übertrager 130 zu dem externen Gerät 140 oder in umgekehrter Richtung übertragen werden. An den Anzeigevorrichtungen 146 kann eine Anzeige von gemessenen Spannungen oder Strömen oder Temperaturen erfolgen.

Wird von dem Übertrager 130 eine Überschreitung einer zulässigen Höchsttemperatur des Temperatursensors 132 festgestellt, so kann dieser beispielsweise über Steuersignale eine automatische Abschaltung oder Parameterveränderung der Pulserzeugung des Pulsgenerators 40 bewirken. Es ist beispielsweise denkbar, eine adaptive oder stufenweise Regelung der Pulserzeugung vorzusehen, mit der bei Annäherung an eine vorgesehene Höchsttemperatur die Pulsleistung und/Pulsfrequenz abgesenkt wird. Alternativ oder bei zu hoher Temperatur zusätzlich kann auch die Pulserzeugung zeitweise unterbleiben, bis wieder eine ausreichend niedrige Temperatur erreicht ist.

Figur 5 zeigt das Gehäuse 22 des Katheters 100 aus der Blickrichtung von unten in Figur 4. Zu sehen ist der spiralförmige Aufbau der Spule 128.

Figur 6 zeigt die Spitze des Katheters 100 in einer Darstellung als Längsschnitt, bei der allerdings sowohl der Katheter 100 als auch die vor und hinter der Zeichenebene liegenden elektrischen Zuleitungen 29 und Anschlußdrähte 136, 138 jeweils im Längsschnitt dargestellt sind.

Die elektrischen Zuleitungen 29 und Anschlußdrähte 136, 138 weisen jeweils eine eine Isolierung 44 auf. Die Zuleitungen 29 sind an dem distalen Kontakt 12 bzw. dem proximalen Kontakt 12b innen angelötet. Das Thermoelement 132, das durch eine Kontaktstelle zwischen dem Nickel-Chrom-Anschlußdraht 136 und dem Nickelanschlußdraht 138 gebildet wird, ist in unmittelbarer Nähe über den Anschlußdraht 136 mit dem Kontakt 12 verbunden. Auf diese Weise wird eine gute Wärmeleitung zwischen dem Kontakt 12 und dem Thermoelement 132 erreicht.

Die elektrischen Zuleitungen 29 und die Anschlußdrähte 136, 138 mit ihren jeweiligen Isolierungen 44 verlaufen innerhalb eines verdickten Bereiches der Wand der Hülle 20 des Katheters 100. Die Schlauchleitung 18 wird wie bei dem Katheter aus Fig. 1 durch die zusätzliche innere, schlauchförmige Schicht 46 in der Hülle 20 des Katheters 100 gebildet.

Der von der inneren Schicht 46 geformte Schlauch endet jenseits der Öffnung 16. Er kann jedoch auch, wie wiederum durch strichpunktierte Linien angedeutet ist, bis in die von dem distalen Kontakt 12 gebildete Kappe hineinragen.

Figur 7 zeigt einen Querschnitt durch den Katheter 100 entlang der Linie VII - VII aus Figur 6. Die Figur zeigt die Anordnung der Zuleitungen 29 und Anschlußdrähte 136, 138 und ihre Isolierungen 44 sowie der die Schlauchleitung 18 bildenden Schicht 46.

Die beschriebene Ausführungsform soll eine mögliche Anordnung und Kontaktierung der elektrischen Zuleitungen 29 sowie des Thermoelements 132 und seiner Anschlußdrähte 136, 138 veranschaulichen und außerdem eine Möglichkeit der Ausführung des Übertragers und des Ports aufzeigen. Selbstverständlich kann der Katheter auch einen von hier abweichenden Aufbau aufweisen. So kann beispielsweise auf die Schicht 46 verzichtet werden. Wahlweise können die Zuleitungen 29 und Anschlußdrähte 136, 138 auch in einem Hohlraum innerhalb der Hülle 20 angeordnet sein. Der elektrische Kontakt 12 kann alternativ ebenfalls in Form eines ringförmigen Bands aufgebaut sein. Selbstverständlich können auch mehr als die gezeigten zwei Kontakte vorgesehen sein.

Aus den beschriebenen Kathetern 10 und 100 erhält man jeweils durch Weglassen der Schlauchleitung 18, der seitlichen Öffnung 16 und des Verbindungselements 64 bzw. der Zuspritzkammer 124 eine Ausführungsform einer Epiduralelektrode eines erfindungsgemäßen Elektrodensystems. Eine daran angepaßte Ausführungsform der Sonde ergibt sich beispielsweise durch Weglassen des Zugangskanals 60 und des Anschlusses 36 an dem Adapter 34.

Die anhand der Fig. 1 bis 3 beschriebenen Ausführungsformen der Elektrode, des Katheters 10 und der Sonden sollen eine mögliche Anordnung und Kontaktierung des Sondenkontakts 53 und des elektrischen Kontaktelements 56 sowie eine mögliche Verbindung zwischen einem Zugangskanal 60 und der Schlauchleitung 18 veranschaulichen und eine Möglichkeit der Ausführung des Ports aufzeigen.

Selbstverständlich kann das Elektrodensystem gemäß der Erfindung auch einen hiervon abweichenden Aufbau aufweisen. So kann beispielsweise neben dem elektrischen Kontakt 12 mindestens ein weiterer elektrischer Kontakt vorgesehen sein, und die elektrischen Kontakte können über getrennte Kontaktelemente mit getrennten Sondenkontakten verbunden sein, die beispielsweise auf unterschiedlichen Höhen angeordnet sind. Dabei kann das Gegenstück 52 entsprechend verlängert sein. Weitere elektrische Verbindungen können beispielsweise für einen im distalen Bereich des Katheters angeordneten Temperatursensor vorgesehen sein, dessen Anschlußleitungen im Katheter angeordnet sind. Dieser kann, ähnlich wie anhand des zweiten Ausführungsbeispiels beschrieben, zur Temperaturüberwachung eingesetzt werden, indem beispielsweise der Adapter 34 die entsprechenden Funktionen des Übertragers 130 und des externen Gerätes 140 übernimmt.

Auch die Ausführung des Kupplungselements der Sonde 30 und der Ankuppelvorrichtung des Katheters 10 als Gewindeansatz 50 und Gewindeelement 51 mit Gegenstück 52 ist lediglich ein mögliches Ausführungsbeispiel. So ist beispielsweise auch ein einklickbarer Verschluß oder eine sonstige mechanische Verankerung denkbar.

## Patentansprüche

1. Elektrodensystem mit einer implantierbaren, flexible Elektrode, insbesondere Epiduralelektrode, mit mindestens einem distalen elektrischen Kontakt (12), wobei ein Teil des Elektrodensystems durch eine Energieübertragungseinrichtung (30, 34; 140) gebildet wird, an die ein externer Generator (40) für Elektrostimulation anschließbar ist, daß die Elektrode eine subkutan implantierbare Basiseinheit (22) aufweist, welche über mindestens eine Zuleitung (29) elektrisch mit dem distalen elektrischen Kontakt (12) verbunden ist, und wobei die Basiseinheit (22) und die Energieübertragungseinrichtung (30, 34; 140) dazu ausgebildet sind, bei geeigneter Anordnung zueinander, wobei die Energieübertragungseinrichtung (30, 34; 140) nicht implantiert wird, elektromagnetische Energie von dem Generator (40) durch die Energieübertragungseinrichtung (30, 34; 140) zur Basiseinheit (22) zu übertragen, und wobei die Basiseinheit (22) einen subkutan implantierbaren Port (26) aufweist, die Energieübertragungseinrichtung (30, 34) eine in den Port (26) einführbare Sonde (30) mit mindestens einem Sondenkontakt (53) aufweist, in dem Port (26) mindestens ein mit dem distalen elektrischen Kontakt (12) verbundenes elektrisches Kontaktelement (56) zur Herstellung einer elektrischen Verbindung mit dem Sondenkontakt (53) angeordnet ist, die Sonde (30) ein Kupplungselement (50) aufweist und der Port (26) eine Ankuppelvorrichtung (51, 52) für das Kupplungselement (50) aufweist.

2. Elektrodensystem nach Anspruch 1, **dadurch gekennzeichnet, daß** die Basiseinheit (22) dazu ausgebildet ist, elektromagnetische Energie als Radiofrequenz auf die Zuleitung (29) zu übertragen.

3. Elektrodensystem nach einem der vorstehenden Ansprüche, **dadurch gekennzeichnet, daß** die Elektrode im distalen Bereich einen Temperatursensor (132) aufweist, der über Anschlußleitungen (136: 138) mit der Basiseinheit (22) verbunden ist.

4. Elektrodensystem nach einem der vorstehenden Ansprüche, **dadurch gekennzeichnet, daß** die Ankuppelvorrichtung (51, 52) ein Gewindeelement (51) aufweist und das Kupplungselement (50) der Sonde (30) einen Gewindeansatz (50) aufweist.

5. Elektrodensystem nach einem der vorstehenden Ansprüche, **dadurch gekennzeichnet, daß** ein Teil einer Wandung des Ports (26) durch ein Septum (28) zum Durchstechen mit einer Nadel (32) gebildet wird.

6. Elektrodensystem nach Anspruch 5, **dadurch gekennzeichnet, daß** das Elektrodensystem eine Nadel (32) umfaßt und daß die Nadel (32) die Sonde (30) aufnimmt und die Sonde (30) aus der Nadel (32) vorschiebbar ist.

7. Elektrodensystem nach einem der vorstehenden Ansprüche, **dadurch gekennzeichnet, daß** die Elektrode in einem implantierbaren, flexiblen Katheter, insbesondere Epiduralkatheter (10; 100), angeordnet ist und daß die Basiseinheit (22) einen subkutan implantierbaren Port (26; 126) aufweist, in dem eine Zugangsöffnung (62) des Katheters (10; 100) angeordnet ist.

8. Elektrodensystem nach Anspruch 7, **dadurch gekennzeichnet, daß** die Sonde (30) einen Zugangskanal (60) für die Zugangsöffnung (62) des Katheters (10) aufweist.

9. Implantierbare, flexible Elektrode, insbesondere Epiduralelektrode, für ein Elektrodensystem gemäß einem der Ansprüche 1 bis 8, mit mindestens einem distalen elektrischen Kontakt (12), wobei die Elektrode eine subkutan implantierbare Basiseinheit (22) aufweist, welche über mindestens eine Zuleitung (29) elektrisch mit dem distalen elektrischen Kontakt (12) verbunden ist, und wobei die Basiseinheit (22) dazu ausgebildet ist, bei geeigneter Anordnung zu einer Energieübertragungseinrichtung (30, 34; 140), welche nicht implantiert wird und an welche ein externer Generator (40) für Elektrostimulation anschließbar ist, elektromagnetische Energie, die von dem Generator (40) durch die Energieübertragungseinrichtung (30, 34; 140) zur Basiseinheit (22) übertragen wird, zu empfangen, und wobei die Basiseinheit (22) einen subkutan implantierbaren Port (26) aufweist, in den eine Sonde (30) der Energieübertragungseinrichtung (30, 34) einführbar ist und in dem mindestens ein mit dem distalen elektrischen Kontakt (12) verbundenes elektrisches Kontaktelement (56) zur Herstellung einer elektrischen Verbindung mit einem Sondenkontakt (53) der Sonde (30) angeordnet ist, und wobei der Port (26) eine Ankuppelvorrichtung (51, 52) für ein Kupplungselement (50) der Sonde (30) aufweist.

10. Implantierbarer, flexibler Katheter, insbesondere Epiduralkatheter (100), mit einer Elektrode nach Anspruch 9, **dadurch gekennzeichnet, daß** die Elektrode in dem Katheter (100) angeordnet ist.

11. Sonde für ein Elektrodensystem gemäß einem der Ansprüche 1 bis 8, die in einen subkutan implantierbaren Port (26) einer subkutan implantierbaren Basiseinheit einer implantierbaren, flexiblen Elektrode einführbar ist und mindestens einen Sondenkontakt (53) zur Herstellung einer elektrischen Verbindung mit einem in dem Port angeordneten elektrischen Kontaktelement (56) aufweist und ein Kupplungselement (50) für eine Ankuppelvorrichtung (51, 52) des Ports (26) aufweist.

## Claims

1. Electrode system having an implantable, flexible electrode, in particular an epidural electrode, having at least one distal electrical contact (12), a part of the electrode system being formed by an energy transmission device (30, 34; 140), to which an external generator (40) can be connected for electrical stimulation, wherein the electrode has a base unit (22) which can be implanted subcutaneously and which is electrically connected to the distal electrical contact (12) by means of at least one supply line (29), and the base unit (22) and the energy transmission device (30, 34; 140) are constructed, with a suitable mutual arrangement and, with the energy transmission device (30, 34; 140) not being implanted, to transmit electromagnetic energy from the generator (40) through the energy transmission device (30, 34; 140) to the base unit (22), the base unit (22) has a port (26) which can be implanted subcutaneously, the energy transmission device (30, 34) has a probe (30) which can be introduced into the port (26) and which has at least one probe contact (53), at least one electrical contact element (56) which is connected to the distal electrical contact (12) for producing an electrical connection to the probe contact (53) is arranged in the port (26), the probe (30) has a coupling element (50) and the port (26) has a coupling device (51, 52) for the coupling element (50).

2. Electrode system according to claim 1, **characterised in that** the base unit (22) is constructed to transmit electromagnetic energy to the supply line (29) as a radio frequency.

3. Electrode system according to either of the preceding claims, **characterised in that** the electrode has in the distal region a temperature sensor (132) which is connected to the base unit (22) by means of connection lines (136; 138).

4. Electrode system according to any one of the preceding claims, **characterised in that** the coupling device (51, 52) has a threaded element (51) and the coupling element (50) of the probe (30) has a threaded attachment (50).

5. Electrode system according to any one of the preceding claims, **characterised in that** a part of a wall of the port (26) is formed by a septum (28) to be *perforated* with a needle (32).

6. Electrode system according to claim 5, **characterised in that** the electrode system comprises a needle (32) and **in that** the needle (32) receives the probe (30) and the probe (30) can be pushed forwards out of the needle (32).

7. Electrode system according to any one of the preceding claims, **characterised in that** the electrode is arranged in an implantable flexible catheter, in particular an epidural catheter (10; 100) and **in that** the base unit (22) has a port (26; 126) which can be implanted subcutaneously and in which an access opening (62) of the catheter (10; 100) is arranged.

8. Electrode system according to claim 7, **characterised in that** the probe (30) has an access channel (60) for the access opening (62) of the catheter (10).

9. Implantable flexible electrode, in particular an epidural electrode, for an electrode system according to any one of claims 1 to 8, having at least one distal electrical contact (12), the electrode having a base unit (22) which can be implanted subcutaneously and which is electrically connected to the distal electrical contact (12) by means of at least one supply line (29), and the base unit (22) being constructed, with appropriate arrangement with respect to an energy transmission device (30, 34; 140), which is not implanted and to which an external generator (40) for electrical stimulation can be connected, to receive electromagnetic energy which is transmitted by the energy transmission device (30, 34; 140) from the generator (40) to the base unit (22), and the base unit (22) having a port (26) which can be implanted subcutaneously and into which a probe (30) of the energy transmission device (30, 34) can be introduced, and in which there is arranged at least one electrical contact element (56) which is connected to the distal electrical contact (12) for producing an electrical connection to a probe contact (53) of the probe (30) and the port (26) having a coupling device (51, 52) for a coupling element (50) of the probe (30).

10. Implantable flexible catheter, in particular an epidural catheter (100), having an electrode according to claim 9, **characterised in that** the electrode is arranged in the catheter (100).

11. Probe for an electrode system according to any one of claims 1 to 8, which can be introduced into a subcutaneously implantable port (26) of a subcutaneously implantable base unit of an implantable flexible electrode and has at least one probe contact (53) for producing an electrical connection to an electrical contact element (56) which is arranged in the port and has a coupling element (50) for a coupling device (51, 52) of the port (26).

## Revendications

1. Système d'électrode avec une électrode souple implantable, en particulier une électrode épidurale, ayant au moins un contact électrique distal (12), **caractérisé en ce qu'**une partie du système d'électrode est formée par des moyens de transmission d'énergie (30, 34 ; 140) auxquels un générateur externe (40) peut être raccordé pour une électrostimulation, que l'électrode comporte une unité de base (22) implantable par voie sous-cutanée qui est électriquement reliée au contact électrique distal (12) par l'intermédiaire d'au moins un fil d'alimentation (29), et dans lequel l'unité de base (22) et les moyens de transmission d'énergie (30, 34 ; 140) sont configurés de manière à, lorsqu'ils sont agencés de manière appropriée l'un par rapport à l'autre, les moyens de transmission d'énergie (30, 34 ; 140) n'étant pas implantés, transmettre à l'unité de base (22) de l'énergie électromagnétique provenant du générateur (40) par l'intermédiaire des moyens de transmission d'énergie (30, 34 ; 140), et dans lequel l'unité de base (22) comporte un port (26) implantable par voie sous-cutanée, les moyens de transmission d'énergie (30, 34) comportent une sonde (30) pouvant être introduite dans le port (26) et munie d'au moins un contact de sonde (53), port (26) dans lequel est agencé au moins un élément de contact électrique (56) relié au contact électrique distal (12) pour établir une connexion électrique avec le contact de sonde (53), la sonde (30) comporte un élément de couplage (50) et le port (26) comporte un dispositif de couplage (51, 52) pour l'élément de couplage (50).

2. Système d'électrode selon la revendication 1, **caractérisé en ce que** l'unité de base (22) est configurée pour transmettre de l'énergie électromagnétique sous forme de radiofréquence au fil d'alimentation (29).

3. Système d'électrode selon l'une quelconque des revendications précédentes, **caractérisé en ce que** l'électrode comporte un capteur de température (132) dans la zone distale, ledit capteur étant relié à l'unité de base (22) par l'intermédiaire de fils de connexion (136 ; 138).

4. Système d'électrode selon l'une quelconque des revendications précédentes, **caractérisé en ce que** le dispositif de couplage (51, 52) comporte un élément fileté (51) et l'élément de couplage (50) de la sonde (30) comporte une embase filetée (50).

5. Système d'électrode selon l'une quelconque des revendications précédentes, **caractérisé en ce qu'**une partie d'une paroi du port (26) est formée par un septum (28) destiné à être piqué avec une aiguille (32).

6. Système d'électrode selon la revendication 5, **caractérisé en ce que** le système d'électrode inclut une aiguille (32) et que l'aiguille (32) reçoit la sonde (30) et la sonde (30) peut être avancée à partir de l'aiguille (32).

7. Système d'électrode selon l'une quelconque des revendications précédentes, **caractérisé en ce que** l'électrode est agencée dans un cathéter souple implantable, en particulier un cathéter épidural (10 ; 100), et que l'unité de base (22) comporte un port implantable par voie sous-cutanée (26 ; 126) dans lequel est agencée une ouverture d'accès (62) du cathéter (10 ; 100).

8. Système d'électrode selon la revendication 7, **caractérisé en ce que** la sonde (30) comporte un canal d'accès (60) pour l'ouverture d'accès (62) du cathéter (10).

9. Electrode souple implantable, en particulière électrode épidurale, pour un système d'électrode selon l'une quelconque des revendications 1 à 8, avec au moins un contact électrique distal (12), l'électrode comportant une unité de base (22) implantable par voie sous-cutanée, qui est électriquement reliée au contact électrique distal (12) par l'intermédiaire d'au moins un fil d'alimentation (29), et l'unité de base (22) étant configurée de manière à, lorsqu'elle est agencée de manière appropriée par rapport à des moyens de transmission d'énergie (30, 34 ; 140) non implantés et pouvant être reliés à un générateur externe (40) pour une électrostimulation, recevoir de l'énergie électromagnétique transmise à l'unité de base (22) par le générateur (40) par l'intermédiaire des moyens de transmission d'énergie (30, 34 ;140), et l'unité de base (22) comportant un orifice (26) implantable par voie sous-cutanée, dans lequel une sonde (30) des moyens de transmission d'énergie (30, 34) peut être introduite et dans lequel est agencé au moins un élément de contact électrique (56) relié au contact électrique distal (12) pour établir une connexion électrique avec un contact de sonde (53) de la sonde (30), et le port (26) comportant un dispositif de couplage (51, 52) pour un élément de couplage (50) de la sonde (30).

10. Cathéter souple implantable, en particulier cathéter épidural (100), avec une électrode selon la revendication 9, **caractérisé en ce que** l'électrode est agencée dans le cathéter (100).

11. Sonde pour un système d'électrode selon l'une quelconque des revendications 1 à 8, ladite sonde pouvant être introduite dans un port (26) implantable par voie sous-cutanée d'une unité de base implantable par voie sous-cutanée d'une électrode souple implantable, et comportant au moins un contact de sonde (53) pour établir une connexion électrique avec un élément de contact électrique (56) agencé dans le port, et comportant un élément de couplage (50) pour un dispositif de couplage (51, 52) du port (26).
